# EUROPEAN PATENT APPLICATION

(11) **EP 2 402 016 A1**
(43) Date of publication of application: **04.01.2012**
(21) Application number: 10167742.5
(22) Date of filing: 29.06.2010
(51) Int. Cl.: A61K 31/7088, C12N 15/115, C07K 16/28

(54) **Aptamers that inhibit interaction between antibody and 1st or 2nd extracellular loop of human beta-1-adrenergic receptor**

(71) Applicant: Charité - Universitätsmedizin Berlin, 10117 Berlin (DE); MAX-DELBRÜCK-CENTRUM FÜR MOLEKULARE MEDIZIN, 13092 Berlin (DE); APTARES AG, 15749 Mittenwalde (DE)
(72) Inventor: Schimke, Ingolf, 12621 Berlin (DE); Haberland, Annekathrin, 10439 Berlin (DE); Kage, Andreas, 10829 Berlin (DE); Wallukat, Gerd, 13129 Berlin (DE); Dahmen, Claudia, 10829 Berlin (DE)
(74) Representative: Gulde Hengelhaupt Ziebig & Schneider

(57) **Abstract**

The present invention is directed to aptamers that are characterized in that it inhibits the agonistic effect of an antibody specific for the 1st or 2nd extracellular loop of human beta-1-adrenergic receptor in a rat cardiomyocyte beating frequency assay with an IC50 of 100 nM or less.

## Description

The immune system forms an essential part of every animal. Mammals make use of its immune system in the defence against microorgansims, in detection and removal of aberrant cells like e.g. tumor cells, and in regeneration of tissue. Thereby the organism relies on two interconnected defence mechanisms, humoral and cellular immunity.

Antibodies, when bound to its antigen are triggers of the humoral immune response. Antibodies can act in multiple ways. Apart from neutralisation of the antigen, antibodies also activate the complement system. Specific binding of antibodies to own cells can activate natural killer cells (NK cells) which are able to facilitate degradation of these cells.

Autoimmune diseases are based on such specific recognition and binding of antibodies to own constituent parts of the body which triggers an immune response against own cells or tissues. Apart from this immunostimulatory effect, such autoantibodies can contribute to the development of pathogenic phenotypes also by other mechanisms. It is well known that autoantibodies can also be specific for the extracellular part of adrenergic G-protein coupled receptors like e.g. beta-1- or beta-2-adrenergic receptors and, upon specific binding, can activate these receptors. The presence of such autoantibodies in an organsim can lead to agonistic effects in the sense of a continuous activation of the respective receptor.

Dilated cardiomyopathy (DCM) is one disease which is based on the presence of such activating autoantibodies binding to extracellular parts of the beta-1-adrenergic receptor, in particular to the 1st or 2nd loop of beta-1-adrenergic receptor. Upon binding of these autoantibodies the receptors are continuously activated (Jahns et al. (2004) Direct evidence for a beta-1-adrenergic receptor-directed autoimmune attack as a cause of idiopathic cardiomyopathy. J.Clin.lnvest. 113, 1419 to 1429).

In recent studies, it could be shown that removal of these autoantibodies from the blood via immunoglobulin adsorption contributes to regeneration of the heart muscle (W.V. Dörffel, S.B. Felix, G. Wallukat, S. Brehme, K. Bestvater, T. Hofmann, F.K. Kleber, G. Baumann, P. Reinke (1997) Short-term hemodynamic effects of immunoadsorption in dilated cardiomyopathy. Circulation 95, 1994-1997 and W.V. Dörffel, G. Wallukat, Y. Dörffel, S.B. Felix, G. Baumann (2004) Immunoadsorption in idiopathic dilated cardiomyopathy, a 3-year follow-up. Int J. Cardiol. 97, 529-534)

However, removal of autoantibodies from body fluids of a patient is costly and comprises the risk of complications. Thus, there is a high need for other modalities to treat cardiomyopathies associated with the presence of autoantibodies specific for the beta-1-adrenergic receptor.

The present invention provides novel compounds suitable for use in treatment and/or diagnosis of diseases associated with the presence of autoantibodies specific for human beta-1-adrenergic receptor in particular for use in treatment of cardiomyopathies.

The present invention provides aptamers characterized in that these aptamers inhibit the agonistic effect of an antibody specific for the 1st or 2nd extracellular loop of human beta-1-adrenergic receptor in a rat cardiomyocyte beating frequency assay with an IC50 of 100 nM or less.

It has surprisingly been found that aptamers of the invention can be used to interfere with the interaction of antibodies, in particular of autoantibodies, specific for the 1st or 2nd extracellular loop of human beta-1-adrenergic receptor with its target. By inhibiting this interaction the aptamers of the invention diminish or even abolish the permanent activation of the beta-1-adrenergic receptor without the need for removal of these antibodies. Thus, the present invention provides novel compounds suitable for use in treatment and/or diagnosis of diseases associated with the presence of autoantibodies specific for human beta-1-adrenergic receptor, in particular for use in treatment and/or diagnosis of cardiomyopathies.

For the purpose of this invention, the term "aptamer" refers to an oligonucleotide that binds specifically and with high affinity to a target molecule, in particular to an antibody which binds to the 1st or 2nd extracellular loop of human beta-1-adrenergic receptor. Under defined conditions, aptamers fold into a specific three dimensional structure which can comprise at least one so called hair-pin structure. The aptamer of the invention preferably exhibits at least one first hair-pin structure comprising a stem region and a loop region. For the purpose of the present invention, the term "hair-pin structure" or "hair-pin" refers to a particular form of a secondary structure of a nucleic acid sequence molecule, preferably of aptamers, which comprises two sequence parts that are complementary to each other and both together form the stem structure. The hair-pin structure comprises an additional sequence part, positioned between the two complementary sequence parts, and forming a loop structure.

The aptamer of the invention comprises or consists of a sequence of nucleic acid molecules, the nucleotides. The aptamer of the invention preferably comprises unmodified and/or modified D- and/or L-nucleotides. According to the common one letter code of nucleic acid bases "C" stands for cytosine, "A" stands for adenine, "G" stands for guanine, and "T" stands for thymine, whereas "U" stands for uracil. If not indicated below to the contrary, the term "nucleotide" shall refer to ribonucleotides and desoxyribonucleotides. Respectively the terms "2'-fluoro-modified nucleotide", "2'-methoxy-modified nucleotide", and/or "2-amino-modified nucleotide" refers to modified ribonucleotides and modified desoxyribonucleotides.

The aptamer of the invention can comprise a nucleic acid sequence of ≥ 7 nucleotides to ≤ 160 nucleotides, preferably of ≥ 12 nucleotides to ≤ 120 nucleotides. In a particular preferred embodiment, the apatmer of the invention comprises or consists of ≥ 44 nucleotides to ≤ 120 nucleotides, more preferably of ≥ 50 nucleotides to ≤ 88 nucleotides, even more preferred of ≥ 60 nucleotides to ≤ 80 nucleotides.

The aptamer of the invention can comprise or consist of a DNA- or an RNA-nucleotide sequence and, thus, can be referred to as DNA-aptamer or RNA-aptamer respectively. It is understood that, if the aptamer of the invention comprises an RNA-nucleotide sequence, within the sequence motifs specified throughout the present invention thymin is replaced by uracil. The RNA-nucleotide sequences of the present invention are identical with the DNA-nucleotide sequences of the invention with the proviso that T is replaced by U. For the sake of conciseness throughout the present invention reference is made solely to explicit DNA-nucleotide sequences. However, it is understood that the respective RNA-nucleotide sequences are also comprised by the present invention.

The use of DNA-aptamers is particularly preferred. DNA-aptamers are usually more stable in plasma than RNA-aptamers.

The aptamers of the invention may comprise a nucleotide sequence containing 2'-modified nucleotides, e.g. 2'-fluoro-, 2'-methoxy- and/or 2'-amino-modified nucleotides. The aptamer of the invention may also comprise a mixture of desoxyribonucleotides, modified desoxyribonucleotides, ribonucleotides and/or modified ribunucleotides.

The aptamer of the invention may comprise modifications. Such modifications encompass e.g. alkylation, i.e. methylation, arylation or acetylation of at least one nucleotide, the inclusion of enantiomers and/or the fusion of aptamers with one or more other nucleotides or nucleic acid sequences. Such modifications may comprise e.g. 5'-PEG- and/or 3'-CAP-modifications. Alternatively or in addition the aptamer of the invention may comprise modified nucleotides, preferably selected from locked-nucleic acids, 2'-fluoro-, 2'-methoxy- and/or 2'-amino-modified nucleotides.

Locked nucleic acids (LNA) represent analogons of the respective RNA nucleotides wherein the conformation has been fixed. Oligonucleotides of locked nucleic acids comprise one or more bicyclic ribonucleosides, wherein the 2'OH group is connected with the C4-carbon atom via a methylen group. Locked nucleic acids exhibit an improved stability versus nucleases compared to the respective unmodified RNA-aptamer counterparts. Also the hybridisation properties are improved which allows for an enhancement of affinity and specificity of the aptamer.

Another preferred modification is the addition of a so called 3'-CAP-, a 5'-CAP-structure and/or of a modified guanosin-nucleotide (e.g. 7-methyl-guanosin) to the 3'- and/or 5'-end of the aptamer. Such a modification of the 3'- and/or 5'-end has the effect that the aptamer is protected from a fast degradation by nucleases.

Alternatively or in adition, the aptamer of the invention can exhibit a pegylated 5'-end. A 5'-PEG modification comprises the addition of at least one polyethylene glycol (PEG) unit, preferably the PEG group comprises 1 to 900 ethylene groups, more preferably from 1 to 450 ethylene groups. In a preferred embodiment, the aptamer comprises linear PEG units with HO-(CH₂CH₂O)ₙ-H, wherein n is an integer of 1 to 900, preferably n is an integer of 1 to 450.

The aptamer of the invention can comprise or consist of a nucleic acid sequence with a phospho-thioate backbone or can be wholly or in part configured as a peptide nucleic acid (PNA).

One advantage of modifying the aptamer of the invention by one of the ways mentioned above is that the aptamer can be stablilized against detrimental influences like e.g. nucleases present in the environment wherein the aptamer is used. Said modifications are also suitable to adapt the pharmacological properties of the aptamer. The modifications preferably do not alter the affinity or specificity of the aptamer.

The aptamer of the invention may also be conjugated to a carrier molecule and/or to a reporter molecule. Carrier molecules comprise such molecules that, when conjugated to the aptamer, prolong the plasma half life of the conjugated aptamer in human plasma, e.g. by enhancing the stability and/or by affecting the excretion rate. One example of a suitable carrier molecule is PEG. Reporter molecules comprise molecules that allow for the detection of the conjugated aptamer. Examples of such reporter molecules are GFP, biotin, cholesterol, dyes like e.g. fluorescence dyes, electrochemically active reporter molecules and/or compounds comprising radioactive residues, in particular radionuclides suitable for PET (positron emission tomography) detection like e.g. ¹⁸F, ¹¹C, ¹³N, ¹⁵O, ⁸²Rb or ⁶⁸Ga. The skilled person is well aware of suitable carrier and reporter molecules and of ways of how to conjugate them to the aptamer of the invention.

The aptamer of the invention is characterized in that it inhibits the agonistic effect of an antibody specific for the 1st or 2nd extracellular loop of human beta-1-adrenergic receptor in a rat cardiomyocyte beating frequency assay with an IC50 of 100 nM or less, preferably with an IC50 of 100 nM to 0,01 nM, more preferably with an IC50 of 10 nM to 0,01 nM, most preferably with an IC50 of 5 nM to 0,01 nM.

The so called rat cardiomyocyte beating frequency assay is a well established assay for detection and characterization of antibodies, e.g. autoantibodies derived from patients, specific for human beta-1-adrenergic receptor. The assay is described in detail in Wallukat et al. (1987) Effects of the serum gamma globulin fraction of patients with allergic asthma and dilated cardiomyopathy on chromotropic beta adrenoceptor function in cultured neonatal rat heart myocytes, Biomed. Biochim. Acta 46, 634 - 639; Wallukat et al. (1988) Cultivated cardiac muscle cells - a functional test system for the detection of autoantibodies against the beta adrenergic receptor, Acta Histochem. Suppl. 35, 145 - 149; and Wallukat et al. (2010) Distinct patterns of autoantibodies against G-protein coupled receptors in Chagas' cardiomyopathy and megacolon. Their potential impact for early risk assessment in asymptomatic Chagas' patients, J. Am. Coll. Cardiol. 55, 463 - 468. Thus, the skilled person is well aware of the nature of this assay and knows how to apply it.

The aptamer of the invention inhibits the agonistic effect of an antibody specific for the 1 st or 2nd extracellular loop of human beta-1-adrenergic receptor in above mentioned assay. The effect of such an antibody is referred to as agonistic, if administration of said antibody leads to a measurable increase in beating frequency, preferably to an increase in beating frequency by at least 2 beats per 15 seconds compared to control without administration of said antibody. Said agonistic effect of the antibody is inhibited by the aptamer of the invention if the increase in beating frequency is reduced, preferably reduced by at least 50 %, more preferably the increase in beating frequency is completely abolished by administration of the aptamer of the invention. The IC50 of said inhibition is calculated as the concentration of the aptamer of the invention in nM at which half of the maximum inhibitory effect of said aptamer is achieved.

In a preferred embodiment the aptamer of the invention not only inhibits the agonistic effect of an antibody specific for the 1st or 2nd extracellular loop of human beta-1-adrenergic receptor, but also specifically binds to said antibody. An aptamer of the invention preferably binds to such an antibody with a higher affinity than said aptamer binds to other targets. Preferably the aptamer of the invention binds to said antibody with an affinity of ≤ 1 pM, more preferably with an affinity of ≤ 500 nM, even more preferably with an affinity of ≤ 100 nM.

The aptamer of the invention inhibits the agonistic effect of an antibody. For the purpose of the present invention, the term "antibody" refers to naturally occuring antibodies, including e.g. autoantibodies in particular autoantibodies of a patient suffering from a disease related to the presence of autoantibodies specific for human beta-1-adrenergic receptor, and modified or genetically engineered antibodies. An autoantibody is an antibody manufactured by the immune system of an organism that is directed against one or more of the organism's own proteins. However, the term antibody is not limited to an antibody with the classical heavy and light chain architecture. The terms "antibody" or "antibodies" as used herein are art-recognized terms and are understood to refer to molecules or active fragments of molecules that bind to given antigens, particularly the terms refer to immunoglobulin molecules and to immunologically active portions of immunoglobulin molecules, i.e molecules that contain a binding site that specifically binds an antigen. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass. The antibody of the invention can be of IgG class or any subclass thereof (IgG1, IgG2, IgG3, IgG4).

A typical antibody structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively.

Antibodies exist as full length intact antibodies or as a number of well-characterized fragments produced by digestion with various peptidases or chemicals. Thus, for example, pepsin digests an antibody below the disulfide linkages in the hinge region to produce F(ab')2, a dimer of Fab which itself is a light chain joined to VH-CH1 by a disulfide bond. The F(ab')2 may be reduced under mild conditions to break the disulfide linkage in the hinge region thereby converting the F(ab')2 dimer into two Fab' monomers. The Fab' monomer is essentially a Fab fragment with part of the hinge region (see, Fundamental Immunology, W. E. Paul, ed., Raven Press, N.Y. (1993), for a more detailed description of other antibody fragments).

While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using recombinant DNA methodologies.

"Antibodies" are intended within the scope of the present invention to include autoantibodies, monoclonal antibodies, polyclonal antibodies, chimeric, single chain, bispecific, simianized, human and humanized antibodies as well as active fragments thereof. Examples of active fragments of molecules that bind to known antigens include separated light and heavy chains, Fab, Fab/c, Fv, Fab', and F(ab')2 fragments, including the products of an Fab immunoglobulin expression library and epitope-binding fragments of any of the antibodies and fragments mentioned above.

The antibody is specific for the 1st or 2nd extracellular loop of human beta-1-adrenergic receptor. An antibody is referred to as being specific for a given antigen or target if said antibody binds to said antigen or target with greater affinity than it does to a structurally different antigen, preferably the antibody binds to said antigen or target with an affinity of ≤ 1 µM. For the purpose of the present invention, the term "human beta-1-adrenergic receptor" refers to human beta-1-adrenergic receptor with the amino acid sequence of SEQ ID No. 6, also available under accession No. NP_000675.1.

In a preferred embodiment, the antibody is specific for the second extracellular loop of human beta-1-adrenergic receptor. In a particularly preferred embodiment the antibody is specific for a particular peptide present within the 2nd extracellular loop of human beta-1-adrenergic receptor. Said peptide comprises or consists of an amino acid sequence with SEQ ID No. 1.

In a preferred embodiment, the antibody specific for the 1st or 2nd extracellular loop of human beta-1-adrenerig receptor, in particular specific for the peptide with SEQ ID No. 1, is a polyclonal goat anti-ADBR1 antibody (ADBR1 = human beta-1-adrenergic receptor) being cross reactive for human, rat, mouse and dog beta-1-adrenergic receptor i.e. the respective homologous protein. Said polyclonal goat anti-ADBR1 antibody is derived by immunization with a peptide consisting of an amino acid sequence with SEQ ID No. 1. Said goat anti-ADBR1 antibody can be purchased from Everest Biotech Ltd, UK, under the order No. EB07133.

In a preferred embodiment, the aptamer of the invention comprises or consists of one of the following nucleic acid sequences:
a) a sequence with one of SEQ ID Nos 2 to 5; or
b) a sequence which is at least to 80% identical to one of the sequences with SEQ ID Nos 2 to 5, preferably a sequence which is at least 90%, more preferably at least 95%, most preferably at least 99% identical to one of the sequences with SEQ ID Nos 2 to 5.

Identity between a nucleic acid sequence and a sequnece with SEQ ID No. 2 to 5 is understood as meaning the identity of the respective sequences over the complete length of the respective sequence with SEQ ID No. 2 to 5. Said identity can be calculated by different ways. Said identity can be calculated using the blastn algoryithm or alternatively with the aid of the GAP program algorithm (Wiskonin Packae Version 10.0, University of Wiskonsin, genetics Computer Group (GCG), Madison, USA), setting the following parameters:

| | |
|---|---|
| Gap weight: | 12 |
| Length weight: | 4 |
| Average match: | 2.912 |
| Average mismatch: | -2.003 |

For example, a sequence which has at least 80% identity with one of SEQ ID No. 2 to 5 is understood as meaning a sequence which, upon comparison with one of SEQ ID Nos 2 to 5 by one of above mentioned program algorithm, exhibits at least 80% homology or identity.

The sequences specified under SEQ ID Nos 2 to 5 comprise nucleic acid sequences that are responsible for a specific inhibition of the interaction between the 1 st or 2nd extracellular loop of human beta-1-adrenergic receptor and an antibody binding specifically to the 1st or 2nd extracellular loop of human beta-1-adrenergic receptor. In particular, the use of one of these sequences within an aptamer leads to an aptamer that inhibits the agonistic effect of an antibody specific for the 1st or 2nd extracellular loop of human beta-1-adrenergic receptor in a rat cardiomyocyte beating frequency assay with an IC50 of 100 nM or less.

In a particularly preferred embodiment, the aptamer of the invention comprises or consists of a nucleic acid sequence with one of SEQ ID Nos 2, 3, 4 or 5.

In a further preferred embodiment, the aptamer of the invention is characterized in that said aptamer in addition inhibits the agonistic effect of an antibody specific for human beta-2-adrenergic receptor in a rat cardiomyocyte beating frequency assay.

The present invention also relates to an aptamer of the invention coupled to a solid support. The skilled person is well aware of techniques and materials which may be used to produce such aptamers coupled to a solid support. In a preferred embodiment, the solid support comprises a solid material that is applicable in medical, biochemical or biological assays. Said solid material comprises polymers that are usually used as support in medical, biochemical or biological assays. In particular the aptamer of the invention may be coupled to a solid support that allows for the use of the resulting product in the manufacturing of a column suitable for apheresis, preferably a column that is suitable for use in an apheresis to remove antibodies specific for the 1st or 2nd extracellular loop of human beta-1-adrenergic receptor from a liquid sample, prefrably from a body fluid.

The aptamer of the invention can be identified and isolated by a novel and inventive combinaton of known techniques. In particular, the aptamer of the present invention can be identified and isolated from a collection of aptamers by selection of such aptamers that exhibit an affinity against an antibody specific for the 1 st or 2nd extracellular loop of human beta-1-adrenergic receptor or a mixture thereof, like e.g. a goat anti-ADBR1 antibody specific for SQ ID No. 1, being cross reactive for human, rat, mouse, and dog beta-1-adrenergic receptor and which can be purchased from Everest Biotech Ltd, UK, under the order No. EB07133, or an polyclonal autoantibody isolated from e.g. DCM patients. Such a selection can be performed by known techniques like e.g. SELEX or SELEX based techniques or by Monolex or Monolex-based techniques (see e.g. US 5,270,163, US 5,723,592, WO 2002/29093 A2). The skilled person is well aware of suitable techniques and of how to successfully apply these. In a subsequent step, the selected aptamers are tested for their capability to inhibit the agonistic effect of an antibody specific for the 1 st or 2nd extracellular loop of human beta-1-adrenergic receptor (the same or a different antibody as the antibody used for selection in the previous step) in a rat cardiomyocyte beating frequency assay. Those aptamers are chosen that exhibit the desired IC50 in above mentioned assay. The sequence of such an aptamer of the invention can be determined again by known methods. The manufacturing or mass production of aptamers with a known sequence is well known in the art and represents a mere routine activity.

Thus, the present invention also relates to a method of isolating an aptamer of the invention comprising the steps:
i) providing an antibody or a mixture of antibodies being specific for the 1st or 2nd extracellular loop of human beta-1-adrenergic receptor, preferably being specific for a peptide with the amino acid sequence of SEQ ID No. 1;
ii) selecting from a collection of aptamers those aptamers that are specific for said antibody or mixture of antibodies;
iii) testing the selected aptamers for their inhibitory effect on the agonistic action of an antibody specific for the 1st or 2nd extracellular loop of human beta-1-adrenergic receptor in a rat cardiomyocyte beating frequency assay; and
iv) selecting those aptamers that exhibit an IC50 of 100 nM or less in the test of step iii).

In a further aspect, the present invention is directed to aptamers of the invention which are isolated by above mentioned method of the invention.

The present invention is also directed to a pharmaceutical composition comprising at least one aptamer of the invention and, optionally, at least one pharmaceutically acceptable excipient. The invention is also directed to a pharmaceutical composition comprising an aptamer of the invention or a mixture of different aptamers of the invention and a pharmaceutically acceptable excipient like e.g. a suitable carrier or diluent.

Preferably the aptamer of the invention is an active ingredient of the pharmaceutical composition and/or is present in an effective amount.

The term "effective amount" denotes an amount of the aptamer of the invention having a prophylactically or therapeutically relevant effect on a disease or pathological conditions. A prophylactic effect prevents the outbreak of a disease. A therapeutically relevant effect relieves to some extent one or more symptoms of a disease or returns to normal either partially or completely one or more physiological or biochemical parameters associated with or causative of the disease or pathological conditions. The respective amount for administering the aptamer of the invention is sufficiently high in order to achieve the desired prophylactic or therapeutic effect. It will be understood by the skilled person that the specific dose level, frequency and period of administration to any particular mammal will depend upon a variety of factors including the activity of the specific components employed, the age, body weight, general health, sex, diet, time of administration, route of administration, drug combination, and the severity of the specific therapy. Using well-known means and methods, the exact amount can be determined by one of skill in the art as a matter of routine experimentation.

In the pharmaceutical composition of the invention at least 20% of the total aptamer content is made of an aptamer of the invention, preferably at least 50%, more preferably at least 75%, most preferable at least 95%.

When used for therapy or prophylaxis, the pharmaceutical composition will generally be administered as a formulation in association with one or more pharmaceutically acceptable excipients. The term "excipient" is used herein to describe any ingredient other than the aptamer of the invention. The choice of excipient will to a large extent depend on the particular mode of administration. Excipients can be suitable carriers and/or diluents.

The pharmaceutical composition of the invention may be administered orally. Oral administration may involve swallowing, so that the composition enters the gastrointestinal tract, or buccal or sublingual administration may be employed by which the composition enters the blood stream directly from the mouth.

Formulations suitable for oral administration include: solid formulations such as tablets; capsules containing particulates, liquids, or powders; lozenges (including liquid-filled); and chews; multi- and nano-particulates; gels; solid solutions; liposomes; films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

For tablet dosage forms, depending on dose, the aptamer of the invention may make up from 0,1 weight % to 80 weight % of the dosage form, more typically from 5 weight % to 60 weight % of the dosage form. In addition to the aptamer of the invention, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

Solid formulations for oral administration may be formulated to be immediate and/or modified release. Modified release formulations include delayed-, sustained-, pulsed-, controlled-, targeted and programmed release.

The pharmaceutical composition of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of pharmaceutical composition of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLApoly(dl-lactic-coglycolic)acid (PGLA) microspheres.

The pharmaceutical composition of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated. Other means of topical administration include delivery by electroporation, iontophoresis, phonophoresis, sonophoresis and microneedle or needle-free (e.g. Powderject(TM), Bioject(TM), etc.) injection. Formulations for topical administration may be formulated to be immediate and/or modified release. Modified release formulations include delayed-, sustained-, pulsed-, controlled-, targeted and programmed release.

The aptamer of the invention and/or the pharmaceutical composition of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins.

For administration to human patients, the total daily dose of the aptamer of the invention and/or the pharmaceutical composition of the invention is typically in the range 0.001 mg to 5000 mg depending, of course, on the mode of administration. For example, an intravenous daily dose may only require from 0.001 mg to 40 mg. The total daily dose may be administered in single or divided doses and may, at the physician's discretion, fall outside of the typical range given herein.

These dosages are based on an average human subject having a weight of about 65 kg to 70 kg. The physician will readily be able to determine doses for subjects whose weight falls outside this range, such as infants and the elderly.

The present invention is also directed to a medical formulation, e.g. one of the formulations mentioned above intended for medical use or application, comprising an aptamer of the invention and/or a pharmaceutical composition of the invention.

The present invention also encompasses a kit comprising an aptamer of the invention, a pharmaceutical composition or a medical formulation of the invention, optionally with written instructions for use and/or with means for administration.

In a further aspect the present invention is directed to an aptamer of the invention, a pharmaceutical composition, a medical formulation or a kit of the invention for treatment and/or diagnosis of a disease, preferably for use in treatment and/or diagnosis of cardiomyopathy, idiopathic cardiomyopathy, dilative cardiomyopathy (DCM), peripartum cardiomyopathy (PPCM), idiopathic cardiomyopathy, Chagas' cardiomyopathy, myocarditis and/or glaucoma.

The terms "treatment" and "therapeutically," as used herein, refer to the act of treating, as "treating" is defined below. As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition.

For treatment and/or diagnosis of a disease, irrespective of the route of administration, the aptamer of the invention is administered at a daily dose per treatment cycle of not more than 20 mg/kg body weight, preferably of not more than 10 mg/kg body weight, more preferably selected from the range of 1 µg/kg to 20 mg/kg body weight, most preferably selected from a range of 0.01 to 10mg/kg body weight.

The aptamer of the invention, when used for treatment or diagnosis of a disease does not necessarily need to be administered to an individual or patient. The therapeutic or diagnostic effect may also be achieved by use of the aptamer of the invention for elimination of antibodies, like e.g. autoantibodies, specific for the 1st or 2nd extracellular loop of human beta-1-adrenergic receptor from the body or from body fluids. Such an elimination may comprise the application of the aptamer of the invention in a setting where the aptamer of the invention is contacted with a body fluid solely ex vivo (e.g. immune adsorption or immune apharesis), so that the aptamer of the invention does not enter the body of the individual or patient to be treated.

The present invention is also directed to the use of an aptamer of the invention or pharmaceutical composition of the invention in the manufacture of a medicament for treatment and/or diagnosis of a disease, preferably of cardiomyopathy, idiopathic cardiomyopathy, dilated cardiomyopathy (DCM), peripartum cardiomyopathy (PPCM), Chagas' cardiomyopathy, myocarditis and/or glaucoma.

In another aspect, the present invention is directed to a method of treatment or diagnosis of a disease, preferably of cardiomyopathy, idiopathic cardiomyopathy, dilated cardiomyopathy (DCM), peripartum cardiomyopathy (PPCM), Chagas' cardiomyopathy, myocarditis and/or glaucoma, wherein an individual in need of such treatment is administered an effective amount of an aptamer of the invention or a pharmaceutical composition of the invention.

In a further aspect, the present invention is directed to the use of an aptamer of the invention for the in vitro detection and/or characterization of antibodies, like e.g. autoantibodies, specific for the 1st or 2nd extracellular loop of human beta-1-adrenergic receptor, preferably for the in vitro detection of antibodies specific for a peptide comprising or consisting of the amino acid sequence with SEQ ID No. 1.

Such a use may comprise the testing of a sample in a rat cardiomyocyte beating frequency assay in the presence and absence of an effective amount of an aptamer of the invention. Depending on the effect of the sample and the aptamer of the invention on the beating frequency, the skilled person can conclude on the presence of antibodies specific for the 1st or 2nd extracellular loop of human beta-1-adrenergic receptor. Data on total or relative quantity of such antibodies in the sample may also be gained as well as on other properties of such antibodies.

The aptamer of the invention can particularly be used in detection and/or characterization of respective antibodies, wherein the antibodies are presented in or derived from a body fluid, preferably a fluid of the human body, more preferably of human blood, plasma, serum, urine, feces, synovial fluid, interstitial fluid, lymph, saliva, sudor, spinal fluid and/or lacrimal fluid. In a preferred embodiment, the body fluid is taken from an individual suffering from or suspected to suffer from a disease, in particular from a cardiomyopathy, dilated cardiomyopathy (DCM), peripartum cardiomyopathy (PPCM), idiopathic cardiomyopathy, Chagas' cardiomyopathy, myocarditis and/or glaucoma.

For detection and/or characterization of such antibodies, the aptamer of the invention may be used in solution or in an immobilized form.

The aptamer of the invention may be used for direct or indirect detection and/or characterization of said antibodies.

In another aspect of the invention, the aptamer of the invention may be used to eliminate in vitro antibodies specific for the 1st or 2nd extracellular loop of the human beta-1-adrenergic receptor from a sample, preferably from a body fluid, more preferably from a fluid of the human body, even more preferably of human blood, plasma, serum, urine, feces, synovial fluid, interstitial fluid, lymph, saliva, sudor, spinal fluid and/or lacrimal fluid. In a preferred embodiment, the body fluid is taken from an individual suffering from or suspected to suffer from a disease, in particular from a cardiomyopathy, dilated cardiomyopathy (DCM), peripartum cardiomyopathy (PPCM), idiopathic cardiomyopathy, Chagas' cardiomyopathy, myocarditis and/or glaucoma.

In Table 1 a concordance list of the sequences referred to under the respective SEQ ID No. is presented.

**Tabelle 1. Concordance list of Seq ID Nos**

| **SEQ ID NO** | **Kind** | **Species** | **Description** |
|---|---|---|---|
| 1 | protein | homo sapiens | epitope of 2nd extracellular loop of human beta-1-adrenergic receptor |
| 2 | nucleic acid | artificial | specific part of aptamer 110 |
| 3 | nucleic acid | artificial | specific part of aptamer 111 |
| 4 | nucleic acid | artificial | whole sequence aptamer 110 |
| 5 | nucleic acid | artificial | whole sequence aptamer 111 |
| 6 | protein | homo sapiens | human beta-1-adrenergic receptor with Accession No NP_000675.1 |
| 7 | nucleic acid | artificial | aptamer 109 |
| 8 | nucleic acid | artificial | control for aptamer 110 |
| 9 | nucleic acid | artificial | control for aptamer 111 |

In the following the invention will be further explained and exemplified by way of examples.

### FIGURES:

- Figure 1: shows the dose dependent agonistic effect of goat-anti-ADBR1 antibody on beat frequency in rat cardiomyocytes (n=6) with a maximum agonistic effect at 3.33 nM.
- Figure 2: shows the inhibitory influence of aptamers of the invention on the agonistic effect of goat anti-ADBR1 antibody on beat frequency in rat cardiomyocytes.
- Figure 3: shows a dose-response curve for the aptamers 110 and 111 with regard to its inhibitory effect of goat anti-ADBR1 antibody on beat frequency in rat cardiomyocytes.
- Figure 4: shows the inhibitory influence aptamers 110 and 111 on the agonistic effect of autoantibody fraction derived from DCM patients on beat frequency in rat cardiomyocytes.
- Figure 5: shows the effect of aptamer 111 on autoantibody and isoprenaline stimulated beat frequency of rat cardiomyocytes.
- Figure 6: shows the inhibitory influence aptamers 110 and 111 on the agonistic effect of autoantibody fraction derived from patients suffering from Chagas' cardiomyoparthy (n=6) and peripartum cardiomyopathy (n=1) on beat frequency in rat cardiomyocytes.
- Figure 7: shows that the effect of selected aptamers as such on the beat frequency of rat cardiomyocytes.
- Figure 8: shows the inhibitory influence of aptamers 110 and 111 on the agonistic effect of autoantibody fraction derived from glaucoma patients on beat frequency in rat cardiomyocytes.

### EXAMPLES:

### Material and methods:

### Identification and quantification of autoantibodies specific for the 1st or 2nd extracellular loop of human beta-1-adrenergic receptor (AAB) using a bioassay of spontaneously beating cultured neonatal rat cardiomyocytes Bioassay principle

For the identification and quantification of the AAB, a bioassay was used which was firstly established for the measurement of AAB against G-protein coupled receptors by Wallukat et al. (Wallukat G, Wollenberger A. Effects of the serum gamma globulin fraction of patients with allergic asthma and dilated cardiomyopathy on chromotropic beta adrenoceptor function in cultured neonatal rat heart myocytes. Biomed Biochim Acta 1987;46:S634-9.) and which we recently described in detail for the measurement of AAB against the adrenergic beta1 and beta2- and the muscarinergic M2-receptors in chronic Chagas disease (Wallukat G, Muñoz Saravia SG, Haberland A, Bartel S, Araujo R, Valda G, Duchen D, Diaz Ramirez I, Borges AC, Schimke I. Distinct patterns of autoantibodies against G-protein-coupled receptors in Chagas' cardiomyopathy and megacolon. Their potential impact for early risk assessment in asymptomatic Chagas' patients. J Am Coll Cardiol. 2010;55:463-8.).

In this bioassay, the chronotropic response of spontaneously beating cultured neonatal rat cardiomyocytes was recorded which is the sum of positive chronotropy caused by stimulating AAB such as the ones targeting adrenergic beta1- and beta2-receptors or the adrenergic alpha1 receptor and negative chronotropy caused by inhibiting AAB such as the ones targeting muscarinergic M2-receptors or the endothelin receptor type A (ETA-receptor) (1 unit of AAB activity = 1 beat/min frequency change).

To differentiate the AAB species with respect to their contribution to the chronotropic response (positive or negative chronotropy), the analysis was conducted in the presence of specific antagonists such as ICI-118.551 for beta2-AAB, atropine for M2-AAB and propranolol for beta1/beta2-AAB. The remaining activity change is caused by AAB except the once which were specifically blocked.

The bioassay can detect and quantify all human serum AAB and other molecules which target receptors on the cell surface whose sequences are homolog to the human receptors (in the case of AAB targeting) and which are linked to a machinery regulating the beating frequency (contractility, chronotropy) of the cells such as the G-protein system.

AAB positivity was defined using cut offs which were calculated based on x ± 3 SD of the AAB levels of more than 100 healthy subjects found in previous studies. For the positive chronotropic response of cultured neonatal rat cardiomyocytes to e.g. beta1-AAB and beta2-AAB, a cut off ≥ 4.0 U and for the negative response to e.g. M2-AAB a cut off ≥ -4.0 U were calculated.

### Cardiomyocyte preparation and culturing

Hearts of 1 to 3 day old rats were removed under sterile conditions and transferred to phosphate buffered saline solution (PBS) containing penicillin/streptomycin. The ventricle tissue was separated, dissected in pieces and washed twice with 10 ml PBS containing penicillin/streptomycin and at last once with PBS only. The ventricle pieces were suspended in 30 ml PBS containing 0.2% trypsin. After incubation for 20 min at 37°C, the trypsination was stopped with 10 ml ice-cold heat-inactivated calf serum. The resulting suspension was centrifuged at 130 x g for 6 min and the pellet transferred to 20 ml of SM20-I medium. For cell count estimation, 100 µl of this suspension was added to 100 µl trypanblou solution. For cell culturing 2.4x 10⁶ cells were suspended in 2.5 ml of glucose containing SM 20-I medium which was equilibrated with humid air and supplemented with 10% heat-inactivated calf serum, 0.1 mU insulin, and 2 µM fluorodeoxyuridine (preventing the overgrowth of the myocytes by non-myocytes), transferred to 12.5-cm² Falcon flasks and cultured as monolayers for 4 days at 37°C. The medium was renewed after two days.

### Bioassay standardization

On the fourth day, the medium was removed and replaced by 2 ml of SM20-I containing 10 % heat-inactivated calf serum and incubated at 37°C for 2 h. Following the incubation, the flasks were transferred onto a heated stage (37°C) of an inverted microscope where 8 synchronously beating fields of the cell layer were marked. The number of beats of each field was counted for 15 sec and averaged using computer assistance based on the program "Imagequant Fourier analysis 100". To use the bioassay for the estimation of the AAB activities, the following criteria must be fulfilled:
1. The basal beating rate must range between 120 and 160 beats/min.
2. Cells stimulated for 5 min with isoprenaline (10 µM) as positive control have to respond with a frequency increase of more than 45 to 50 beats/min.
3. The cells must respond to a further positive control, a commercial polyclonal antibody against the second extracellular loop of the human beta1-adrenergic receptor generated in goats (Goat anti ADRB1; EB07133; Everest Biotech Ltd, Oxfordshire, UK). After incubation of the cells for 1 hour with the commercial autoantibody the beating frequency must increase by 20 beats/min and its chronotropic effects must strongly be blocked by propranolol antagonizing both beta1 and beta2 receptors and by bisoprolol specifically antagonizing the beta1 receptor.

When the bioassay fulfills these criteria, the differences between the basal beating rate and the rate after the addition of the agonist and/or the autoantibodies (delta beating rate) is very similar from day to day even if the basal pulsating rate differs. Therefore the delta beating rate is used to express the stimulating and depressing activity (positive and negative chronotropic effect) of sera containing the different AAB. For the delta beating rate in the presence of isoprenaline and the commercial beta1-AAB, a day to day variation of < 15 % was estimated.

### Preparation of serum samples for AAB identification and differentiation and AAB activity measurement

1 ml of control and patient serum and 660 µl saturated ammonium sulfate solution were mixed (final concentration 40 % ammonium sulphate) and incubated for 18 hours at 4°C. After centrifugation for 15 min at 6,000 x g, the pellet was re-suspended in 750 µl PBS, mixed with 750 µl saturated ammonium sulfate solution (final concentration 50 % ammonium sulfate) and centrifuged again. After this, the pellet was suspended in 700 µl PBS and dialyzed against the 100 fold volume of PBS. The resulting IgG fraction can be stored at -20° C until measurement.

### Measurement of human serum AAB

In cell cultures prepared and cultured as described above, the basal beating rate of 8 fields of the cell layer was counted. The addition of specific blocker/s such as atropine for blocking the M2-receptors of the cells excluded interferences by e.g. M2-receptors and enabled the identification and measurement of the patient AAB. For this purpose patient IgG was added (dilution 1:20) to the assay medium and incubated for 1 hour. Then the beating rate of the same fields as used for the estimation of the basal rate was counted to calculate the delta beating rates.

Further receptor blockers were added and combined in order to identify and separate the single AAB activities. The process of serum AAB identification was followed by the process of AAB characterization (epitope finding). For this purpose the measurements were repeated in the presence of epitope mapping peptide libraries which competed with the receptor about the AAB binding prohibiting the beating frequency change, when corresponding to the epitope.

### Testing the specificity of aptamers for single AAB

Aptamers targeting AAB and inhibiting the functionality of these AAB with respect to the change of the beating frequency of the rat cardiomyocytes were identified and tested in the bioassay.

For this purpose the aptamers were added to the AAB (or serum sample) prior the addition of this complex mixture to the cell cultivation medium. After an incubation of about 1 hour the beating frequency of the cells was registered and the delta beating rate compared to the basal beating frequency was calculated. Selecting specific well characterized sera/AAB enabled to prove the specificity of the aptamers for one or several AAB.

The dose-response relationship was investigated at a constant AAB concentration, varying the aptamer concentration.

Using the commercially available AAB, specific for the adrenergic beta 1 receptor and possessing a known quantity/beating frequency increase ratio, enabled to quantify the dose-response of the specific anti-beta 1 receptor AAB-aptamer. The dose-response was in the equimolar range.

### Selection of the oligonucleotides (aptamers)

Oligonucleotid aptamers are short single or double stranded DNA or RNA sequences which are selected according to their affinity towards the target of interest. The aptamers used against the AAB (goat-anti ADRB1) are single stranded DNA (dsDNA) sequences. The Monolex technology was exploited for their selection. The target was goat anti-ADBR1 antibody specific for SQ ID No. 1, which can be purchased from Everest Biotech Ltd, UK, under the order No. EB07133.

### Production of the oligonucleotides (aptamers)

The aptamers of the invention can be single stranded DNA sequences. Aptamers, including chemically substituted aptamers, can be synthesized using any oligonucleotide synthesis techniques known in the art, including solid phase oligonucleotide synthesis techniques (see, e.g., Froehler et al, Nucl. Acid Res., 14:5399- 5467 (1986) and Froehler et al, Tet. Lett., 27:5575-5578 (1986)) and solution phase methods, such as triester synthesis methods (see, e.g., Sood et al, Nucl. Acid Res., 4:2557 (1977) and Hirose et al, Tet. Lett., 28:2449 (1978))". The aptamers of the invention were synthesized using the solid phase oligonucleotide synthesis technique. Running all the described methods and procedures enabled us to find aptamers of the present invention.

### Results:

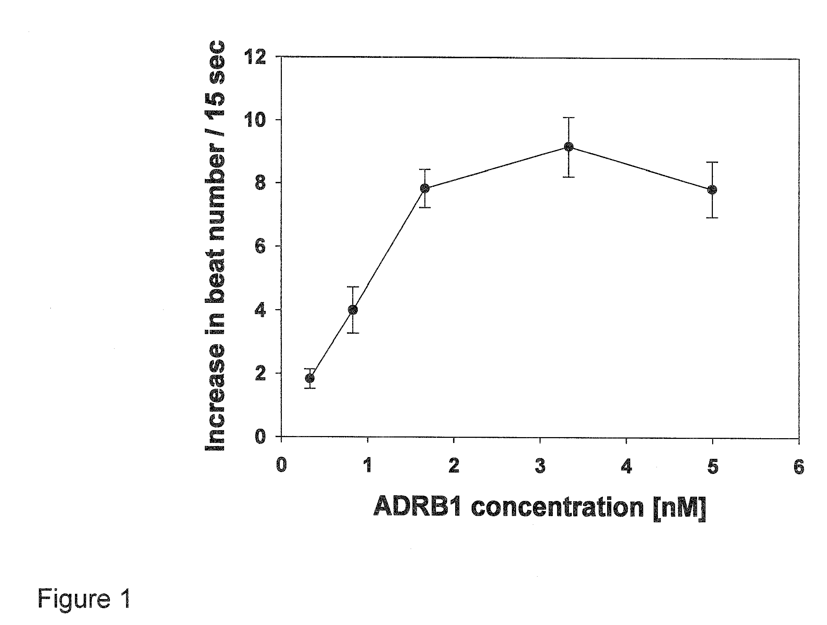

As demonstrated in Figure 1, the agonistic effect of goat-anti-ADRB1 was tested on spontaneously beating neonatale rat cardiomyocytes which were isolated and cultured as described under material and methods. Briefly: after trypsinization of small ventricle pieces from hearts of 1-3 day old rats the myocytes were isolated an cultured in 12.5 cm² flasks using SM20-I cell medium supplemented with heat inactivated calf serum, insulin, and fluorodeoxyuridine. The increase of the beating frequency after drug/antibody application was standardized in independent experiments using isoprenaline and goat-anti-ADRB1. The drug/antibody induced increase of the beating frequency was set in relation to the drug/antibody free spontaneous beating frequency (basal beating frequency), expressed as delta beats/15 sec. Here an increasing amount of goat-anti-ADRB1was added onto the cells in culture. After incubation of the cells with the goat-anti-ADRB1 antibody for 1 hr the beating rate was counted and set in relation to the basal beating rate. The goat-anti-ADRB1 concentration which induced the maximum agonistic effect (3.33 nM) was chosen for further experiments testing the inhibitory effect of the aptamers. Independent epitope mapping experiments revealed the second extracellular loop of the ß1-receptor as the target for goat-anti-ADRB1.

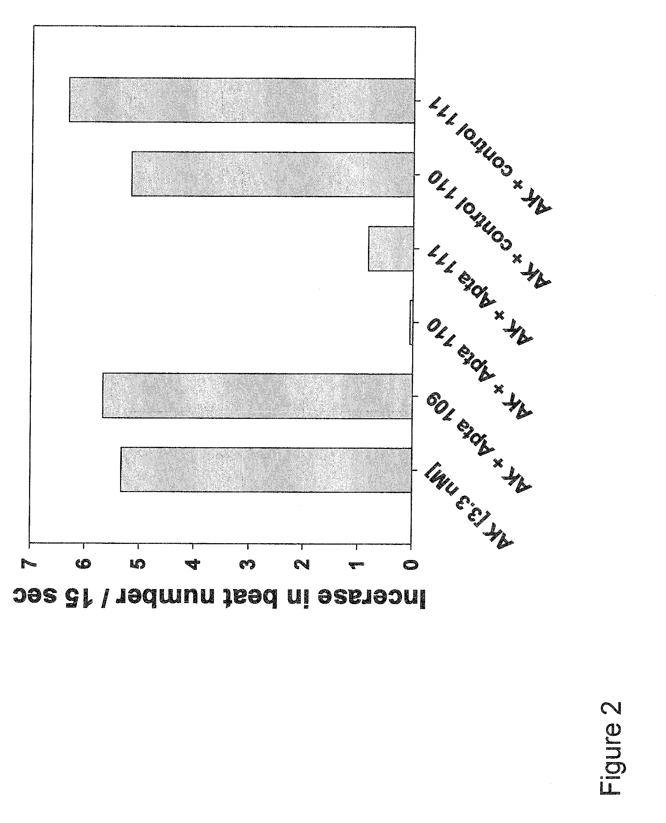

As shown in Figure 2, the aptamers of the invention Apta 110 and 111 exhibit a strong inhibition of the agonistic effect of goat anti-ADBR1 antibody on beat frequency in rat cardiomyocytes at a concentration of 25 nM, whereas control aptamers did not reveal any significant effect. For this experiment the aptamers were added to goat-anti-ADRB1 diluted 1:10 in phosphate buffered saline and incubated for 10 min before this premix was added onto the spontaneously beating cardiomyocytes and incubated for 1 hr. The final concentrations in the cell cultivating medium are 3.3 nM and 25 nM for goat-anti-ADRB1 and the aptamers, respectively. Afterwards the beating frequency was recorded and set into relation to the basal beating frequency which was recorded before the addition of the antibody/aptamer mixture (basal beating frequency). Apta 109, control 110, and control 111 are aptamers which served for control. Control 110 and control 111 are the scrambled controls of aptamer 110 and 111. Aptamer Apta 110 and 111 were able to inhibit the agonistic effect of goat-anti-ADRB1 at the chosen concentration of 25 nM.

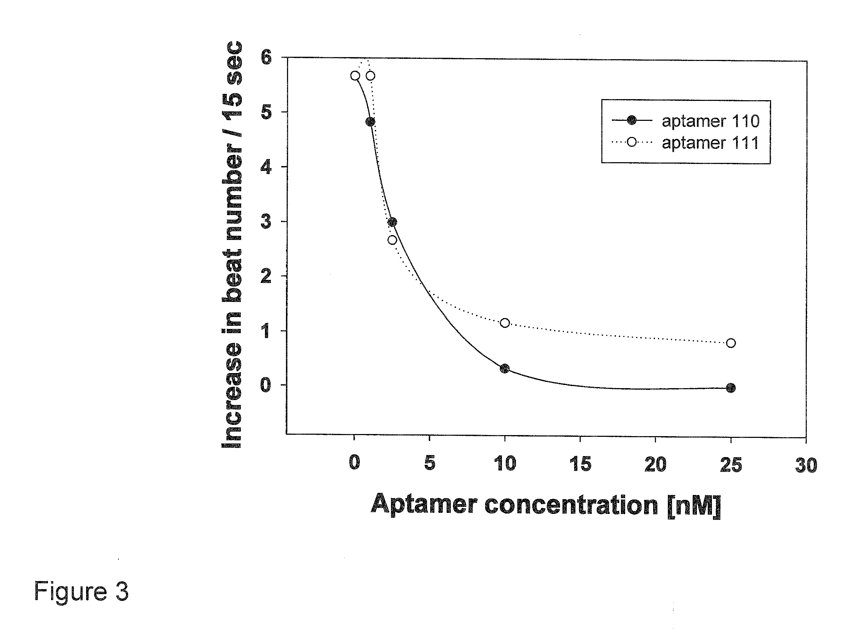

As demonstrated in Figure 3, a dose-response curve could be established for the aptamers of the invention Apt 110 and Apt 111. For this experiment the aptamers at the distinct concentration were added to goat-anti-ADRB1diluted 1:10 in phosphate buffered saline and incubated for 10 min before this premix was added onto the spontaneously beating cardiomyocytes an incubated for 1 hr. The final concentration of the goat-anti-ADRB1 antibody in the cell cultivating medium was 3.3 nM. After the incubation the beating frequency was recorded and set into relation to the basal beating frequency which was taken before the mixture was added. Aptamer 110 caused a complete inhibition of the agonistic effect of goat-anti-ADRB1 while with the aptamer 111 at the investigated concentrations a residual agonistic effect was observable. The IC 50 was within the equimolar range between aptamer and antibody.

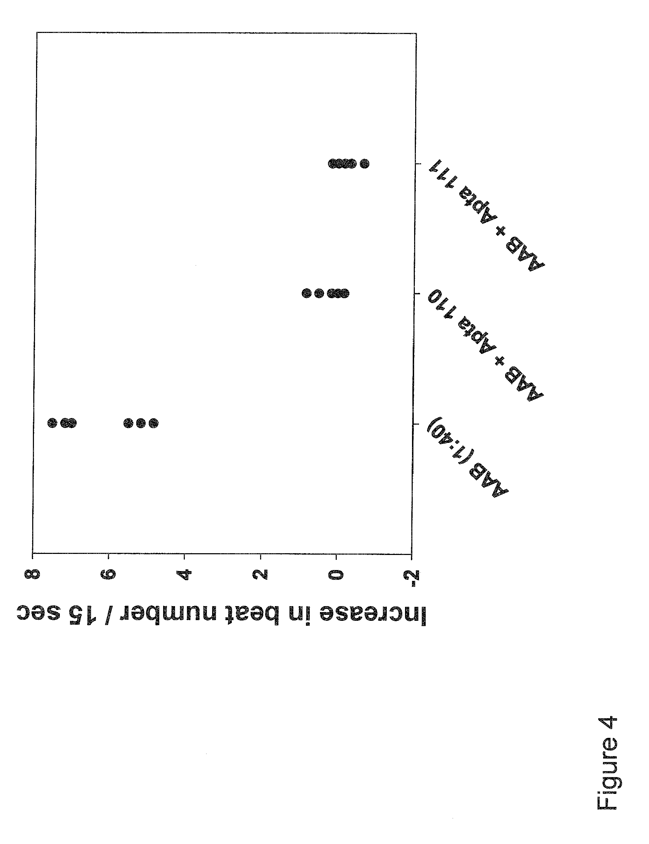

As shown in Figure 4, the aptamers of the invention Apt 110 and Apt 111 proved to be efficacious also in inhibition of the agonistic effect of autoantibody fraction derived from DCM patients on beat frequency in rat cardiomyocytes. For this experiment 2 µl of a 10 µM aptamer solution was added to 50 µl of the patient serum-IgG-fraction (IgG preparation see under materials and methods) and incubated for several minutes before this premix was added onto the spontaneously beating cardiomyocytes and incubated again for 1 hr. The final dilution of the IgG sample in the cell cultivating medium was 1:40 (50 µl IgG fraction in 2 ml cell cultivating medium). After the incubation the beating frequency was recorded and set into relation to the basal beating frequency which was taken before the mixture was added.

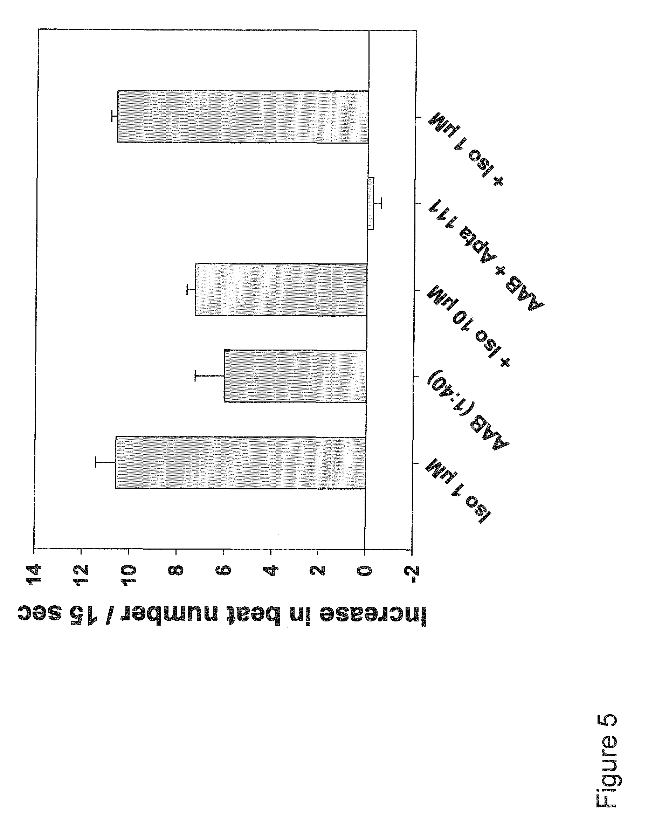

In Figure 5, evidence is provided that the aptamer of the invention is a blocking aptamer that binds to the antibody and prevents the antibody thereby from exerting its agonistic effect on the antigen of the antibody. The first column shows the agonistic effect of 1 µM isoprenaline (n=2). The second column shows the agonistic effect of patient autiantibody (IgG dilution 1:40, n=4). The third column shows the combination of the agonistic effects of the patient autioantibodies (IgG dilution 1:40) and 10 µM isopenaline (n=3). Here it is clearly to see that isoprenaline cannot develop its full agonistic effect which is a sign that the agonistic antibody blocks the adrenergic ß1-receptor; isoprenaline does not have its free access to the receptor. The fourth column shoes the inhibitory effect of 25 nM aptamer Apta 111 on the patient autoantibody (final dilution 1:40, pretreatment see figure legend 4, n=4)). The fifth column shows again the combined addition of patient autoantibody and aptamer Apta 111 but additionally containing 1 µM isoprenaline (n=3). Here isoprenaline can develop its full agonistic response. This is a clear sign that the aptamer-pretreated autoantibody is not able to interfere with the adrenergic ß1-receptor. The receptor molecule is free, showing its free accessibility for the chemical agonist isoprenaline.

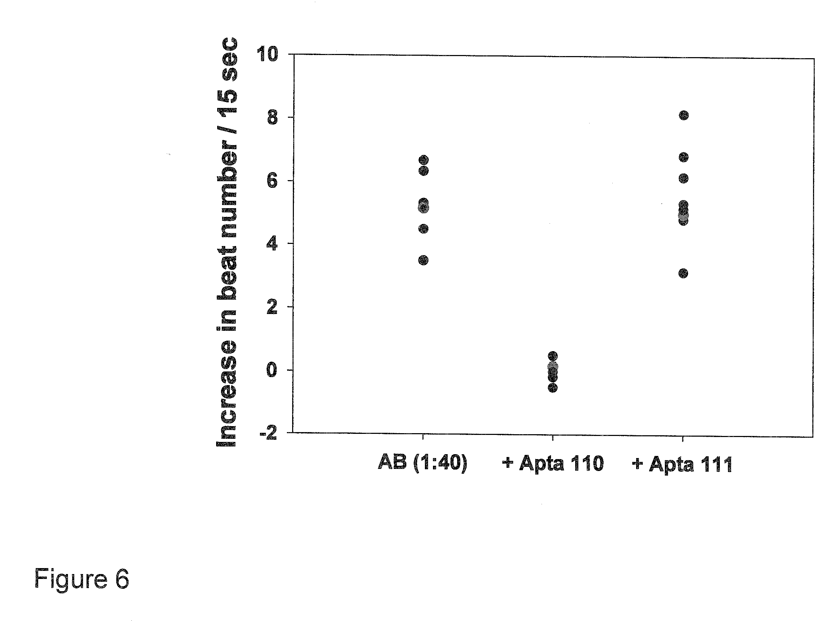

As demonstrated in Figure 6, the aptamers of the invention are also effective with regard to autoantibodies derived from patients suffering from Chagas' cardiomyopathy and peripartum cardiomyopathy. While aptamer Apta 110 showed a strong inhibitory activity at a concentration of 10 nM, aptamer Apta 111 did not. For this experiment 2 µl of a 10 µM aptamer solution was added to 50 µl of the patient serum-IgG-fraction (IgG preparation see under materials and methods) and incubated for several minutes before the premix was added onto the spontaneously beating cardiomyocytes and incubated again for 1 hr. The final dilution of the IgG sample in the cell cultivating medium was 1:40 (50 µl IgG fraction in 2 ml cell cultivating medium). After the incubation the beating frequency was recorded and set into relation to the basal beating frequency which was taken before the mixture was added.

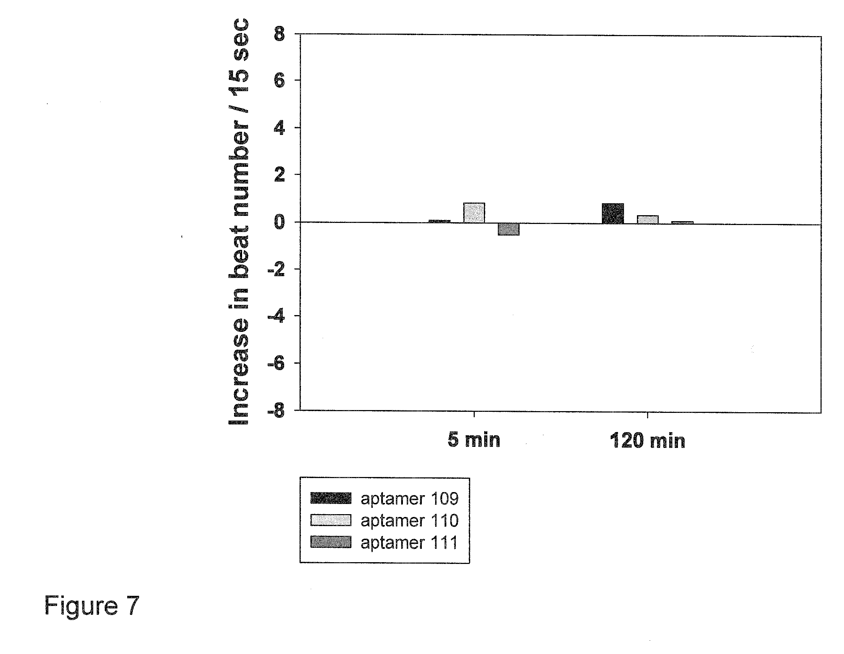

As shown in Figure 7, the aptamers of the invention when administered alone do not show any significant effect. For this experiment 2 µl of a 10 µM aptamer solution was added onto the spontaneously beating cardiomyocytes and incubated for 2 hrs. 5 min and 2 hrs after the incubation the beating frequency was recorded and set into relation to the basal beating frequency which was taken before the aptamers were added.

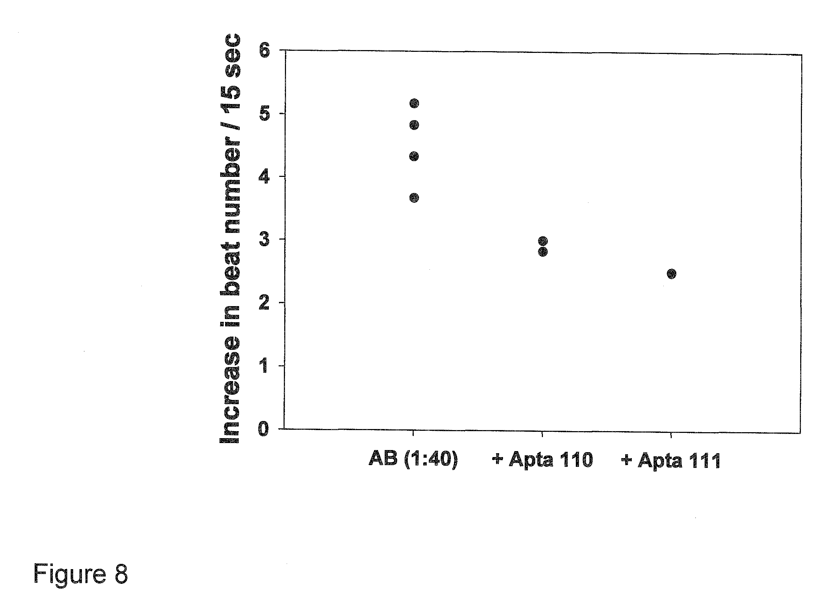

As shown in Figure 8, the aptamers of the invention are also effective with regard to autoantibodies derived from glaucoma patients. For this experiment 2 µl of a 10 µM aptamer solution was added to 50 µl of the patient serum-IgG-fraction (IgG preparation see under materials and methods) and incubated for several minutes before this premix was added onto the spontaneously beating cardiomyocytes and incubated again for 1 hr. The final dilution of the IgG sample in the cell cultivating medium was 1:40 (50 µl IgG fraction in 2 ml cell cultivating medium). After the incubation the beating frequency was recorded and set into relation to the basal beating frequency which was taken before the mixture was added.

### Summary:

Summarising the experiments we can see the following main facts:
1. The commercially available autoantibody goat-anti-ADRB1 is comparable to the DCM-specific patient autoantibody, targeting the DCM-specific epitope of the second extracellular loop of the adrenergic ß1-receptor. ADRB1 increases the beating frequency of spontaneously beating neonatale rat cardiomyocytes, comparable to the patient autoantibody (agonistic effect). The optimum concentration was determined (Fig. 1).
2. Both aptamers of interest (aptamer Apta 110 and aptamer Apta 111) were able to inhibit the agonistic effect of goat-anti-ADRB1 (Fig. 2). The IC50 was in the equimolar range to the antibody (Fig. 3).
3. Both aptamers of interest (aptamer Apta 110 and aptamer Apta 111) were able to inhibit the agonistic effect of DCM-specific patient autoantibodies (Fig. 4).
4. Both aptamers show partial inhibitory effects on ß2-specific autoantibodies of Glaucoma patients at the chosen concentration (Figure 8).
5. Figure 5 shows very clearly that the aptamer-modified autoantibody is not able to bind onto the receptor. A chemical agonist (isoprenaline) is very well able to develop its agonistic effects despite the presence of the autoantibody when the autoantibody is pretreated with the aptamer. At the absence of the aptamer, isoprenaline can not fully develop its agonistic activity because of the receptor binding of the antibody.
6. The aptamers themselves did not show any effects on the beating frequency of the neonatale rat cardiomyocytes (Fig.6).

## Claims

1. Aptamer **characterized in that** it inhibits the agonistic effect of an antibody specific for the 1st or 2nd extracellular loop of human beta-1-adrenergic receptor in a rat cardiomyocyte beating frequency assay with an IC50 of 100 nM or less.

2. Aptamer of claim 1, wherein the aptamer exhibits an IC50 of 100 nM to 0,01 nM.

3. Aptamer of one of the preceding claims, wherein the antibody is specific for a peptide with the amino acid sequence of SEQ ID No. 1.

4. Aptamer of one of the preceding claims, wherein the aptamer comprises or consists of one of the following nucleic acid sequences:
a) a sequence with one of SEQ ID Nos 2 to 5; or
b) a sequence which is at least to 80% identical to one of the sequences with SEQ ID Nos 2 to 5.

5. Aptamer of one of the preceding claims, wherein the aptamer comprises or consists of a nucleic acid sequence with SEQ ID No. 2, 3, 4 or 5.

6. Aptamer of one of the preceding claims, wherein the aptamer is a DNA-Aptamer.

7. Aptamer of one of the preceding claims, wherein the aptamer is coupled to a solid support.

8. Pharmaceutical composition comprising at least one aptamer of one of the claims 1 to 7 and a pharmaceutically acceptable excipient.

9. Kit comprising at least one aptamer of one of the claims 1 to 7.

10. Aptamer of one of claims 1 to 7, pharmaceutical composition of claim 8 or kit of claim 9 for use in treatment and/or diagnosis of a disease.

11. Aptamer, pharmaceutical composition or kit of claim 10 for use in treatment and/or diagnosis of cardiomyopathy, dilated cardiomyopathy (DCM), peripartum cardiomyopathy (PPCM), idiopathic cardiomyopathy, Chagas' cardiomyopathy, myocarditis and/or glaucoma.

12. Use of an aptamer of one of claims 1 to 7 for the in vitro detection and/or characterization of antibodies specific for the 1st or 2nd extracellular loop of human beta-1-adrenergic receptor.

13. Use of claim 12, wherein the antibodies are present in or derived from a body fluid, preferably a fluid of the human body, more preferably of human blood, plasma, serum, urine, feces, synovial fluid, interstitial fluid, lymph, saliva, sudor, spinal fluid and/or lacrimal fluid.

14. Use of claim 13, wherein the body fluid is taken from an individual suffering from or suspected to suffer from cardiomyopathy, dilated cardiomyopathy (DCM), peripartum cardiomyopathy (PPCM), idiopathic cardiomyopathy, Chagas' cardiomyopathy, myocarditis and/or glaucoma.

15. Method of isolating an aptamer of one of claims 1 to 7 comprising the steps:
i) providing an antibody or a mixture of antibodies being specific for the 1st or 2nd extracellular loop of human beta-1-adrenergic receptor;
ii) selecting from a collection of aptamers those aptamers that are specific for said antibody or mixture of antibodies;
iii) testing the selected aptamers for their inhibitory effect on the agonistic action of an antibody specific for the 1 st or 2nd extracellular loop of human beta-1-adrenergic receptor in a rat cardiomyocyte beating frequency assay; and
iv) selecting those aptamers that exhibit an IC50 of 100 nM or less in the test of step iii).


SEQUENCE LISTING

<110> Charite - Universitätsmedizin Berlin
Max-Delbrück-Centrum für Molekulare Medizin
AptaRes AG

<120> Aptamers that inhibbit interaction between antibody and 1st or
2nd extracellular loop of human beta-1-adrenergic receptor

<130> P07789EP

<160> 9

<170> PatentIn version 3.3

<210> 1
<211> 13
<212> PRT
<213> homo sapiens

<400> 1

Glu Ser Asp Glu Ala Arg Arg Cys Tyr Asn Asp Pro Lys
1               5                   10

<210> 2
<211> 21
<212> DNA
<213> artificial

<220>
<223> specific part of aptamer 110

<400> 2
acagtaaccg cgtgaggtcg a 21

<210> 3
<211> 21
<212> DNA
<213> artificial

<220>
<223> specific part of aptamer 111

<400> 3
ggaaatagat ctctagcgtg g 21

<210> 4
<211> 63
<212> DNA
<213> artificial

<220>
<223> aptamer 110

<400> 4
tacgactcac tatagggatc cacagtaacc gcgtgaggtc gagaattccc tttagtgagg 60

gtt 63

<210> 5
<211> 63
<212> DNA
<213> artificial

<220>
<223>  aptamer 111

<400>  5
tacgactcac tatagggatc cggaaataga tctctagcgt gggaattccc tttagtgagg      60

gtt                                                                   63


<210>  6
<211>  477
<212>  PRT
<213>  homo sapiens

<400>  6

Met Gly Ala Gly Val Leu Val Leu Gly Ala Ser Glu Pro Gly Asn Leu
1               5                   10                  15

Ser Ser Ala Ala Pro Leu Pro Asp Gly Ala Ala Thr Ala Ala Arg Leu
            20                  25                  30

Leu Val Pro Ala Ser Pro Pro Ala Ser Leu Leu Pro Pro Ala Ser Glu
        35                  40                  45

Ser Pro Glu Pro Leu Ser Gln Gln Trp Thr Ala Gly Met Gly Leu Leu
    50                  55                  60

Met Ala Leu Ile Val Leu Leu Ile Val Ala Gly Asn Val Leu Val Ile
65                  70                  75                  80

Val Ala Ile Ala Lys Thr Pro Arg Leu Gln Thr Leu Thr Asn Leu Phe
                85                  90                  95

Ile Met Ser Leu Ala Ser Ala Asp Leu Val Met Gly Leu Leu Val Val
            100                 105                 110

Pro Phe Gly Ala Thr Ile Val Val Trp Gly Arg Trp Glu Tyr Gly Ser
        115                 120                 125

Phe Phe Cys Glu Leu Trp Thr Ser Val Asp Val Leu Cys Val Thr Ala
    130                 135                 140

Ser Ile Glu Thr Leu Cys Val Ile Ala Leu Asp Arg Tyr Leu Ala Ile
145                 150                 155                 160

Thr Ser Pro Phe Arg Tyr Gln Ser Leu Leu Thr Arg Ala Arg Ala Arg
                165                 170                 175

Gly Leu Val Cys Thr Val Trp Ala Ile Ser Ala Leu Val Ser Phe Leu
            180                 185                 190

Pro Ile Leu Met His Trp Trp Arg Ala Glu Ser Asp Glu Ala Arg Arg
        195                 200                 205

Cys Tyr Asn Asp Pro Lys Cys Cys Asp Phe Val Thr Asn Arg Ala Tyr
    210                 215                 220

Ala Ile Ala Ser Ser Val Val Ser Phe Tyr Val Pro Leu Cys Ile Met
225                 230                 235                 240

Ala Phe Val Tyr Leu Arg Val Phe Arg Glu Ala Gln Lys Gln Val Lys
                245                 250                 255

Lys Ile Asp Ser Cys Glu Arg Arg Phe Leu Gly Gly Pro Ala Arg Pro
            260                 265                 270

Pro Ser Pro Ser Pro Ser Pro Val Pro Ala Pro Ala Pro Pro Pro Gly
        275                 280                 285

Pro Pro Arg Pro Ala Ala Ala Ala Ala Thr Ala Pro Leu Ala Asn Gly
    290                 295                 300

Arg Ala Gly Lys Arg Arg Pro Ser Arg Leu Val Ala Leu Arg Glu Gln
305                 310                 315                 320

Lys Ala Leu Lys Thr Leu Gly Ile Ile Met Gly Val Phe Thr Leu Cys
                325                 330                 335

Trp Leu Pro Phe Phe Leu Ala Asn Val Val Lys Ala Phe His Arg Glu
            340                 345                 350

Leu Val Pro Asp Arg Leu Phe Val Phe Phe Asn Trp Leu Gly Tyr Ala
        355                 360                 365

Asn Ser Ala Phe Asn Pro Ile Ile Tyr Cys Arg Ser Pro Asp Phe Arg
    370                 375                 380

Lys Ala Phe Gln Gly Leu Leu Cys Cys Ala Arg Arg Ala Ala Arg Arg
385                 390                 395                 400

Arg His Ala Thr His Gly Asp Arg Pro Arg Ala Ser Gly Cys Leu Ala
                405                 410                 415

Arg Pro Gly Pro Pro Pro Ser Pro Gly Ala Ala Ser Asp Asp Asp Asp
            420                 425                 430

Asp Asp Val Val Gly Ala Thr Pro Pro Ala Arg Leu Leu Glu Pro Trp
        435                 440                 445

Ala Gly Cys Asn Gly Gly Ala Ala Ala Asp Ser Asp Ser Ser Leu Asp
    450                 455                 460

Glu Pro Cys Arg Pro Gly Phe Ala Ser Glu Ser Lys Val
465                 470                 475

<210>  7
<211>  63
<212>  DNA
<213>  artificial

<220>
<223>  aptamer 109

<400>  7
tacgactcac tatagggatc caaattaagt gtataacggt cagaattccc tttagtgagg      60

gtt                                                                   63


<210>  8
<211>  63
<212>  DNA
<213>  artificial

<220>
<223>  control aptamer

<400>  8
tacgactcac tatagggatc caaggcatcg aatccggatg gcgaattccc tttagtgagg      60

gtt                                                                   63


<210>  9
<211>  63
<212>  DNA
<213>  artificial

<220>
<223>  control aptamer

<400>  9
tacgactcac tatagggatc catgtaaggc atggatcagt gcgaattccc tttagtgagg      60

gtt                                                                   63